# EUROPEAN PATENT SPECIFICATION

(11) **EP 2 139 513 B1**
(45) Date of publication and mention of the grant of the patent: **05.09.2012**
(21) Application number: 08738393.1
(22) Date of filing: 28.03.2008
(51) Int. Cl.: A61K 39/00, A61K 39/29, A61K 9/50, A61K 9/51

(54) **POLYMER PARTICLES BASED VACCINE**
IMPFSTOFF AUF BASIS VON POLYMERTEILCHEN
VACCIN À BASE DE PARTICULES POLYMÈRES

(30) Priority: 28.03.2007 IN DE06782007
(43) Date of publication of application: 06.01.2010
(73) Proprietor: National Institute of Immunology, New Delhi 110067 (IN)
(72) Inventor: PANDA, Amulya, Kumar, New Delhi 110 067 (IN); VIBHU, Kanchan, New Delhi 110 076 (IN)
(74) Representative: Dilg, Haeusler, Schindelmann Patentanwaltsgesellschaft mbH
(86) International application number: PCT/IN2008/000198
(87) International publication number: WO 2008/117313

(56) References cited:
- WO-A-00/12125
- CHONG C S W ET AL: "Enhancement of T helper type 1 immune responses against hepatitis B virus core antigen by PLGA nanoparticle vaccine delivery" JOURNAL OF CONTROLLED RELEASE, ELSEVIER, AMSTERDAM, NL, vol. 102, no. 1, 20 January 2005 (2005-01-20), pages 85-99, XP004744260 ISSN: 0168-3659 cited in the application
- THOMASIN C ET AL: "Tetanus toxoid and synthetic malaria antigen containing poly(lactide)/poly(lactide-co-glycolide) microspheres: importance of polymer degradation and antigen release for immune response" JOURNAL OF CONTROLLED RELEASE, ELSEVIER, AMSTERDAM, NL, vol. 41, no. 1, 1 August 1996 (1996-08-01), pages 131-145, XP004037578 ISSN: 0168-3659
- RAGHUVANSHI RAJEEV S ET AL: "Improved immune response from biodegradable polymer particles entrapping tetanus toxoid by use of different immunization protocol and adjuvants" INTERNATIONAL JOURNAL OF PHARMACEUTICS, AMSTERDAM, vol. 245, no. 1-2, 1 October 2002 (2002-10-01), pages 109-121, XP002306394 ISSN: 0378-5173
- YING M ET AL: "A single administration of tetanus toxoid in biodegradable microspheres elicits T cell and antibody responses similar or superior to those obtained with aluminum hydroxide" VACCINE, BUTTERWORTH SCIENTIFIC. GUILDFORD, GB, vol. 13, no. 7, 1 January 1995 (1995-01-01), pages 683-689, XP004057594 ISSN: 0264-410X
- GUTIERRO I ET AL: "Size dependent immune response after subcutaneous, oral and intranasal administration of BSA loaded nanospheres" VACCINE, BUTTERWORTH SCIENTIFIC. GUILDFORD, GB, vol. 21, no. 1-2, 22 November 2002 (2002-11-22), pages 67-77, XP004393287 ISSN: 0264-410X cited in the application
- KANCHAN ET AL: "Interactions of antigen-loaded polylactide particles with macrophages and their correlation with the immune response" BIOMATERIALS, ELSEVIER SCIENCE PUBLISHERS BV., BARKING, GB, vol. 28, no. 35, 9 October 2007 (2007-10-09), pages 5344-5357, XP022290574 ISSN: 0142-9612

## Description

### FIELD OF INVENTION

The present invention provides a vaccine composition comprising polymer particles comprising an effective amount of antigen for use in the treatment of a disease involving the induction of a combined humoral and cell mediated immune response.

### BACKGROUND OF THE INVENTION

The current vaccine formulations elicit either humoral or cell-mediated immune response depending on the mode of delivery and adjuvant used. Although previous work in immunological delivery research has achieved delivery of strong antigens to elicit an immune response by bioneutralizing antibodies (humoral response), they fail to elicit cytotoxic T lymphocyte (CTL) response. For many infectious diseases both bioneutralizing antibody and activated T cell mediated responses are desired for immunity. For this very reason, polymer particle based delivery systems are being much sought for their application in single dose vaccines and different therapeutic applications. Such systems can be utilized to develop highly useful, effective vaccines and vaccine delivery system.

Polymer particle based vaccine delivery systems provide a viable alternative to multi-dose immunization schedule for many infectious diseases where neutralizing antibody titers provide protective immunity (Cleland JL. Single-administration vaccines: controlled-release technology to mimic repeated immunizations, Trends Biotechnol 1999; 17:25-29). Particles, particularly made from poly lactide-co-glycolide (PLGA) or PLA, not only work as a delivery system but also provide adjuvant activity (Jiang W, Gupta RK, Deshpande, MC, Schwendeman SP. Biodegradable poly (lactic-co-glycolic acid) microparticles for injectable delivery of vaccine antigens. Adv Drug Deliv Rev 2005; 57:391-410; O'Hagan DT, Singh M. Microparticles as vaccine adjuvants and delivery systems. Expert Rev Vaccines 2003; 2:269-83). These polymeric particulate delivery systems have the capacity to present the antigen both by MHC class I (MHC I) and MHC class II (MHC II) pathway and thus can activate both humoral and cellular response (O'Hagan DT, Singh M. Microparticles as vaccine adjuvants and delivery systems. Expert Rev Vaccines 2003;2:269-83.; Men Y, Audran R, Thomasin C, Eberl G, Demotz S, Merkle HP, et al. MHC class I- and class II-restricted processing and presentation of microencapsulated antigens. Vaccine 1999;17:1047-56.; Carcaboso AM, Hernandez RM, Igartua M, Rosas JE, Patarroyo ME, Pedraz JL. Potent, long lasting systemic antibody levels and mixed Th1/Th2 immune response after nasal immunization with malaria antigen loaded PLGA microparticles, Vaccine 2004; 22:1423-32). Efficient targeting of particulate antigen to the APCs has been reported as a major factor contributing towards the generation of immune response, which requires that the particle size should be between 1-10 µm (Tabata Y, Ikada Y. Macrophage phagocytosis of biodegradable microspheres composed of L-lactic acid/glycolic acid homo- and copolymers. J Biomed Mater Res 1988;22:837-58.; Eldridge JH, Staas JK, Meulbroek JA, McGhee JR, Tice TR, Gilley RM. Biodegradable microspheres as a vaccine delivery system. Mol Immunol 1991;28:287-94.; Thiele L, Rothen-Rutishauser B, Jilek S, Wunderli-Allenspach H, Merkle HP, Walter E. Evaluation of particle uptake in human blood monocyte-derived cells in vitro. Does phagocytosis activity of dendritic cells measure up with macrophages? J Control Release 2001; 76:59-71). Nevertheless, efficient primary antibody responses have been observed with much larger and diverse range of polymer particles having size bigger than that of APCs (Hilbert AK, Fritzsche U, Kissel T. Biodegradable microspheres containing influenza A vaccine: immune response in mice. Vaccine 1999;17:1065-73.; Tabata Y, Inoue Y, Ikada Y. Size effect on systemic and mucosal immune responses induced by oral administration of biodegradable microspheres, Vaccine 1996; 14:1677-85, Shi L, Caulfield MJ, Chern RT, Wilson RA, Sanyal G, Volkin DB. Pharmaceutical and immunological evaluation of a single-shot hepatitis B vaccine formulated with PLGA microspheres. J Pharm Sci 2002;91:1019-35). Immune responses from many polymer entrapped antigens, reveal that micron sized range particles promote humoral response whereas nanoparticles (<1 µm) promote cellular response (Gutierro I, Hernandez RM, Igartua M, Gascon AR, Pedraz JL. Size dependent immune response after subcutaneous, oral and intranasal administration of BSA loaded nanospheres. Vaccine 2002;21:67-77; Harding CV, Song R. Phagocytic processing of exogenous particulate antigens by macrophages for presentation by class I MHC molecules. J Immunol 1994;153:4925-33.; Chong CS, Cao M, Wong WW, Fischer KP, Addison WR, Kwon GS, et al. Enhancement of T helper type 1 immune responses against hepatitis B virus core antigen by PLGA nanoparticle vaccine delivery. J Control Release 2005; 102:85-99). As the humoral and cellular immune responses complement each other, size of the polymeric particulate delivery system influences both the strength and quality of immune response.

It is widely accepted that polymeric particle based vaccine delivery formulations are more immunogenic than soluble antigens *in vivo.* However, there has been much ambiguity and debate over whether this effect can be explained solely by the enhanced uptake of the particles by the professional APCs and more so over the size of particles that can be engulfed by the APCs for antigen presentation to initiate an effective immune response. It has been reported that while non-phagocytic eukaryotic cells can internalize particles < 1 µm in size (Rejman J, Oberle V, Zuhorn IS, Hoekstra D. Size-dependent internalization of particles via the pathways of clathrin- and caveolae-mediated endocytosis. Biochem J 2004; 377:159-69), the professional phagocytic APCs- dendritic cells (DCs) and macrophages-can internalize and present particulate antigens of size >1 µm ( Shen Z, Reznikoff G, Dranoff G, Rock KL. Cloned dendritic cells can present exogenous antigens on both MHC class I and class II molecules, J Immunol 1997; 158:2723-30). Most of these studies used polystyrene particles and there are enough evidences that surface chemistry of particles does influence the uptake rate (Oh YK, Swanson JA, Different fates of phagocytosed particles after delivery into macrophage lysosomes. J Cell Biol 1996; 132:585-93; Tomazic-Jezic VJ, Merritt K, Umbreit TH, Significance of the type and the size of biomaterial particles on phagocytosis and tissue distribution, J Biomed Mater Res 2001;55:523-29.; Foged C, Brodin B, Frokjaer S, Sundblad A. Particle size and surface charge affect particle uptake by human dendritic cells in an in vitro model. Int J Pharm 2005; 298:315-22) thus a clear picture on size based cellular uptake is never observed. PLGA or PLA based nanoparticles are extensively taken up by non-phagocytic eukaryotic cells, macrophages and DCs (Lutsiak ME, Robinson DR, Coester C, Kwon GS, Samuel J. Analysis of poly(D,L-lactic-co-glycolic acid) nanosphere uptake by human dendritic cells and macrophages in vitro. Pharm Res 2002;19:1480-87.; Panyam J, Labhasetwar V. Dynamics of endocytosis and exocytosis of poly(D,L-lactide-co-glycolide) nanoparticles in vascular smooth muscle cells. Pharm Res 2002;20:212-20), however such clear uptake studies with large sized polymer particles have not been shown conclusively (Walter E, Dreher D, Kok M, Thiele L, Kiama SG, Gehr P, et al. Hydrophilic poly(DL-lactide-co-glycolide) microspheres for the delivery of DNA to human-derived macrophages and dendritic cells. J Control Release 2001; 76:149-68; Jones BG, Dickinson PA, Gumbleton M, Kellaway IW. The inhibition of phagocytosis of respirable microspheres by alveolar and peritoneal macrophages, Int J Pharm 2002; 236:65-79, Peyre M, Fleck R, Hockley D, Gander B, Sesardic D, In vivo uptake of an experimental microencapsulated diphtheria vaccine following sub-cutaneous immunisation, Vaccine 2004; 22:2430-37). As the size of PLA/PLGA particles changes from nanometer range to > 1 µm, drastic reduction in uptake of polymer particles by cells is observed (Desai MP, Labhasetwar V, Amidon GL, Levy RJ. Gastrointestinal uptake of biodegradable microparticles: effect of particle size. Pharm Res 1996; 13:1838-45; Horisawa E, Kubota K, Tuboi I, Sato K, Yamamoto H, Takeuchi H, et al. Size-dependency of DL-lactide/glycolide copolymer particulates for intra-articular delivery system on phagocytosis in rat synovium, Pharm Res 2002; 19:132-39) and 0. 5 µm has been suggested as the cut off size for efficient phagocytosis Foster KA, Yazdanian M, Audus KL. Microparticulate uptake mechanisms of in vitro cell culture models of the respiratory epithelium. J Pharm Pharmacol 2001;53:57-66.; Lai SK, Hida K, Man ST, Chen C, Machamer C, Schroer TA, et al. Priveleged delivery of polymer nanoparticles to the perinuclear region of live cells via a non-clathrin, non-degradative pathway. Biomaterials 2007;28:2876-84.; Hirota K, Hasegawa T, Hinata H, Ito F, Inagawa H, Kochi C, et al. Optimum conditions for efficient phagocytosis of rifampicin-loaded PLGA microspheres by alveolar macrophages. J Control Release 2007; 119:69-76). It is quite possible that bigger sized particles are localized in the cell membrane (Lacasse FX, Filion MC, Phillips NC, Escher E, McMullen JN, Hildgen P. Influence of surface properties at biodegradable microsphere surfaces: effects on plasma protein adsorption and phagocytosis. Pharm Res 1998; 15:312-17) and deliver antigen into phagosomes which have the capacity to present the antigen via MHC II pathway (Ramachandra L, Song R, Harding CV. Phagosomes are fully competent antigen-processing organelles that mediate the formation of peptide:class II MHC complexes, J Immunol 1999; 162:3263-72).

Chong and colleagues (Chong, C.S.W. et al. (2005) J. Contr. Release 102, 85-99) reported vaccines for inducing a TH1 immune response against hepatitis B infections. The vaccines employed were formulated as antigen-loaded nanoparticles having a diameter of about 300 nm. Thomasin and colleagues (Thomasin, C. et al. (1996) J. Contr. Release 41, 131-145) described poly(lactide)/poly(lactide-co-glycolide) microspheres for tetanus toxoid and malaria antigen delivery. In particular, two different classes of microspheres were prepared: one in the range of 1-10 µm and the other in the range of 10-100 µm.

Finally, Raghuvanshi and colleagues (Raghuvanshi, R.S. et al. (2001) Int. J. Pharmaceut. 245, 109-121) reported a vaccine formulation comprising poly(lactide)/poly(lactide-co-glycolide) particles entrapping tetanus toxoid. It is shown that employing a mixture of nanoparticles and microparticles results in early and high antibody titers in the animal model tested, that is, in a robust TH2 immune response.

The PCT publication WO0012125A1 describes a method of inducing a polarized TH1 response by parenteral administration of microparticle containing antigen entrapped or encapsulated by a biodegradable polymer. The document further describes a method for inducing a polarized TH2 response by parenteral administration of nanoparticles containing antigen entrapped or encapsulated by a biodegradable polymer.

### SUMMARY OF THE INVENTION

The present invention provides a vaccine composition for use in the treatment of a disease or clinical manifestation involving the induction of a combined TH1 and TH2 immune response, the vaccine composition comprising an effective amount of antigen encapsulated in polymeric particles, wherein a combination of nanoparticles and microparticles, said polymeric particles comprise wherein the nanoparticle induces cellular response i.e Cytotoxic T lymphocyte (CTL) response or TH1 immune response and the microparticle induces humoral response or TH2 response by bioneutralizing antibodies.

For inducing a combined humoral cell mediated immune response in a subject, an effective amount of a vaccine composition, as defined herein, comprising an antigen encapsulated in polymeric particles, wherein said polymeric particles comprises a combination of nanoparticles and microparticles, may be administered to a subject.

### BRIEF DESCRIPTION OF ACCOMPANYING DRAWINGS

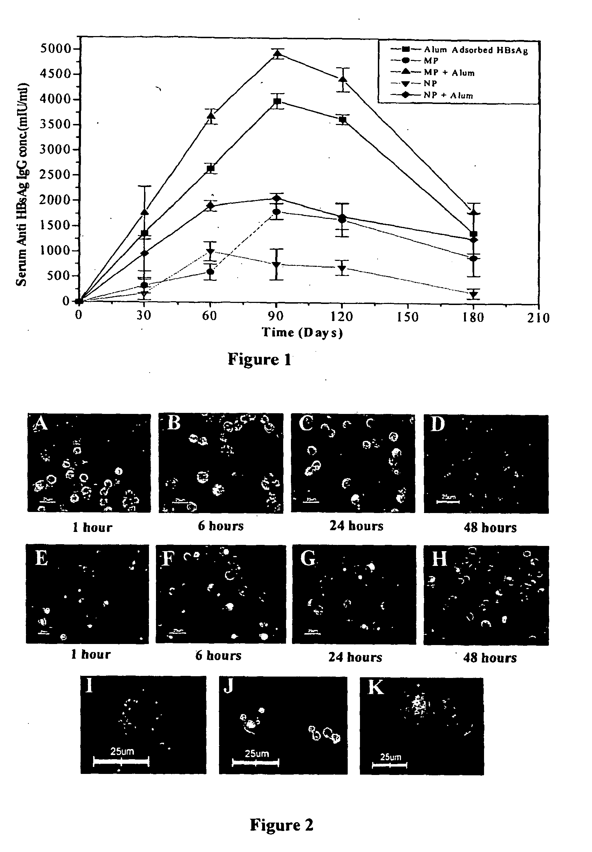

Figure 1: Shows size based anti-HBsAg IgG antibody response from HBsAg entrapped PLA particles. (■) Alum absorbed HBsAg, (●) Microparticles, (▲) Microparticle with alum, (▼) Nanoparticles, and (◆) Nanoparticles with alum. Error bars represent mean ± standard deviation (n = 8).
Figure 2: Demonstrates particle uptake in murine macrophage cell line J774A.1 using different sized 6-coumarin entrapped PLA particles *in vitro* at various time points. (A-D) Nanoparticles uptake in murine macrophage cell line J774A.1. (E-H) Microparticles uptake in murine macrophage cell line J774A.1. Each bar represents 25 µm.

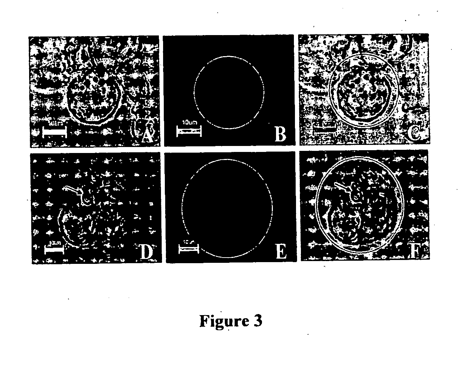

Figure 3: Demonstrates Fluorescence quenching. A, B and C show uptake of FITC-BSA entrapped nanoparticles in macrophages *in vitro* at 24 hours. D, E and F show uptake of FITC-BSA entrapped microparticles in macrophages *in vitro* at 24 hours. Each bar indicates 10 µm.

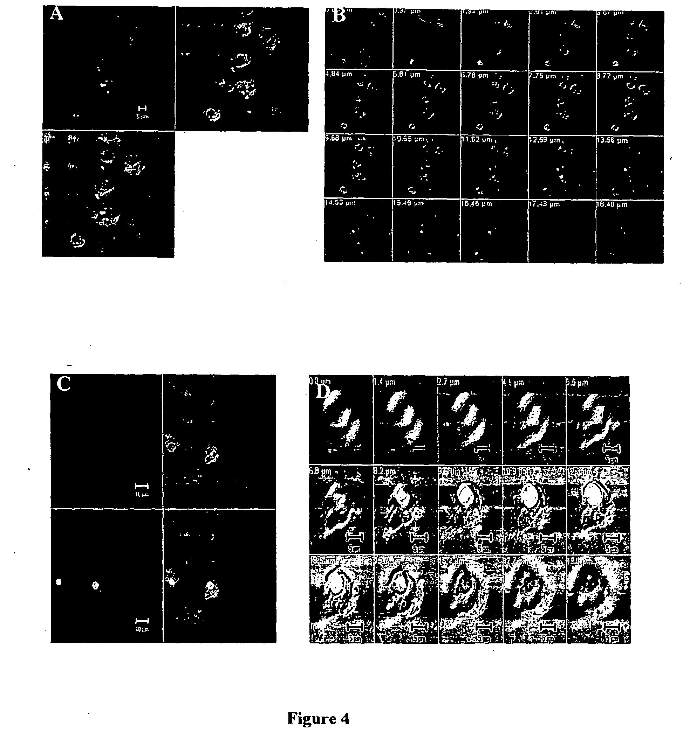

Figure 4: CLSM images showing intracellular uptake and localization of 6-coumarin entrapped PLA nanoparticles and surface attachment of microparticles in murine macrophage cell line J774A.1 *in vitro.* (A) An overview of 6-coumarin entrapped nanoparticles phagocytosed by macrophages *in vitro* at 24 hours is shown. (B) Optical sections (z-sections) of the same macrophage showing phagocytosed nanoparticles at different sections. Each optical section represents a particular plane of focus and nanoparticles have different plane of focus indicating that they are internalized completely. (C) An overview of 6-coumarin based microparticle adhering to a macrophage *in vitro* at 24 hours is shown. (D) Optical sections (z-sections) of the same macrophage showing an adherent microparticle. The data shown here is a representative of a minimum of three individual experiments done at same experimental conditions. Each bar represents 5 µm.

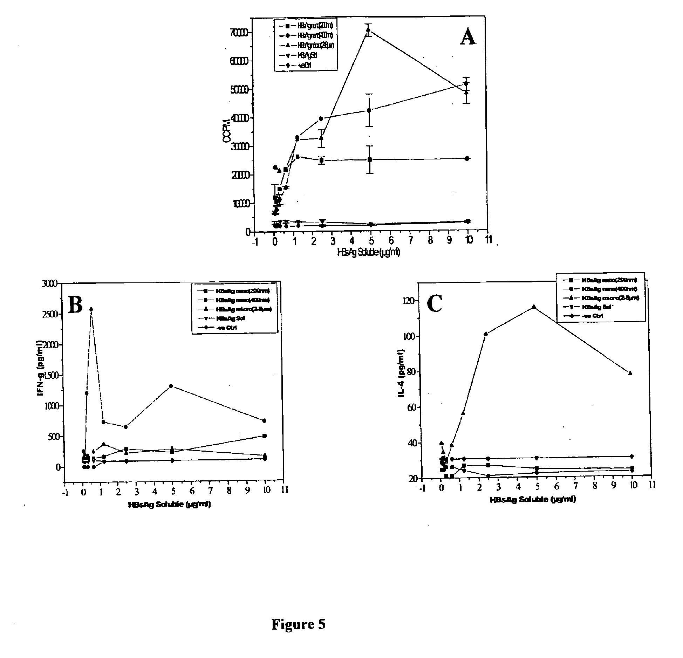

Figure 5 (A) T cell proliferation assay of mice immunized with HBsAg entrapped PLA microparticles and nanoparticles. (B, C) Cytokine assay from the culture supernatants of T cell proliferation assay (A) for the analysis of mouse IFN-γ (B) and mouse IL-4 (C). Error bars represent mean ± standard deviation of triplicate wells.

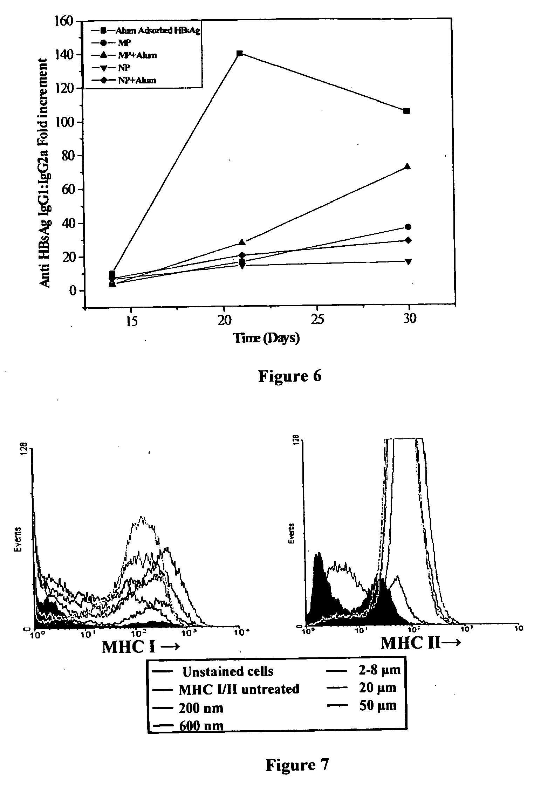

Figure 6. Groups of animals (n=8) immunized with either nanoparticles or microparticles with or without alum as a single dose; or with standard dose of alum adsorbed HBsAg containing 1µg of HBsAg. Serum samples were collected at specific time intervals and analyzed for anti-HBsAg IgG1 and IgG2a antibody titers. The data was expressed as a ratio between IgG1 and IgG2a absorbance values.
Figure 7. Analysis of surface MHC class I levels (left) and MHC class II levels (right) on APCs (macrophages) treated with different sized HBsAg particles at 24h *in vitro.* Particle size is indicated by color bars as: (-) 200 nm; (-) 600 nm; (-) 2-8 µm; (-) 20 µm; (-) 50 µm; (-) untreated cells; and (-) control.

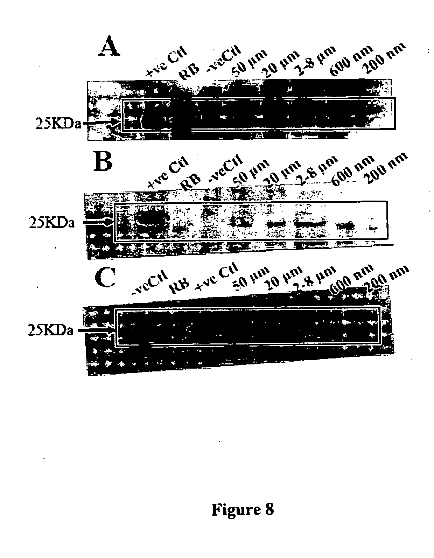

Figure 8 Western blot analysis showing continuous release of HBsAg from microparticles into APCs resulting in its higher accumulation with time. Each lane depicts the intracellular HBsAg content after treatment with a particular size of HBsAg entrapped PLA particles. Untreated cell lysates were taken as negative control (-ve ctl) whereas soluble HBsAg (2-3 µg) was taken as positive control (+ve ctl). RB indicates lane for Rainbow marker.

### DETAILED DESCRIPTION OF THE INVENTION

The present invention provides a vaccine composition for use in the treatment of a disease or clinical manifestation involving the induction of a combined TH1 and TH2 immune response, the vaccine composition comprising an effective amount of antigen encapsulated in polymeric particles, wherein a combination of nanoparticles and microparticles said polymeric particles comprise wherein the nanoparticle induces cellular response i.e cytotoxic T lymphocyte (CTL) response or TH1 immune response and the microparticle induces humoral response or TH2 response by bioneutralizing antibodies.

In one embodiment, the present invention provides a vaccine composition (or vaccine loaded polymer particle delivery system) as defined herein above that is used as a single-shot vaccine formulation for antigens which would otherwise require multiple injections to elicit an immune response.

The vaccine composition as defined herein above comprises a combination of nanoparticles and microparticles , wherein the nanoparticle size ranges from 200-600 nm and microparticle size ranges from 2-8 µm_{.}

The vaccine composition provided in the present invention is useful against various diseases where a combined humoral and cellular immune response is required for such as malaria, tuberculosis, hepatitis, diphtheria, tetanus, cancer and human immunodeficiency virus infection.

The vaccine composition disclosed in the present invention can be useful against other diseases such as AIDS-AIDS related complex, chickenpox, common cold- Influenza (Flu), dengue fever, foot and mouth disease, hepatitis, herpes simplex, HPV, Lassa fever, measles, mumps, poliomyelitis, rabies, SARS, Smallpox, viral encephalitis, viral gastroenteritis, viral meningitis, viral pneumonia, West Nile disease and Yellow fever.

The vaccine composition disclosed in the present invention can be useful against other bacterial diseases such as Anthrax, Bacterial Meningitis, Botulism, Brucellosis, Cat Scratch Disease, Cholera, Diphtheria, Epidemic Typhus, Gonorrhea, Impetigo, Leprosy (Hansen's. Disease), Listeriosis, Rheumatic Fever; Nocardiosis, Pertussis (Whooping Cough), Plague, Pneumococcal pneumonia, Psittacosis, Q fever, Rocky Mountain Spotted Fever (RMSF), Salmonellosis, Scarlet Fever, Shigellosis, Syphilis, Tetanus, Trachoma, Tuberculosis, Tularemia, Typhoid Fever, Typhus and Urinary Tract Infections.

The vaccine composition disclosed in the present invention can be useful against other parasitic diseases such as African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, Trichomoniasis and Trypaonsomiasis.

The vaccine composition disclosed in the present invention can be useful against other fungal diseases such as Aspergillosis, Blastomycosis, Candidiasis, Coccidioidomycosis, Cryptococcosis, Histoplasmosis and Tinea pedis.

The vaccine composition disclosed in the present invention can be useful against diseases such as transmissible spongiform encephalopathy, Bovine spongiform encephalopathy and Creutzfeldt-Jakob disease.

The vaccine composition of the present invention for use in the treatment of a disease or clinical manifestation involving the induction of a combined TH1 and TH2 immune response comprises an antigen encapsulated in polymeric particles, wherein said polymeric particles comprise a combination of nanoparticles, and microparticles wherein the microparticles induce a humoral immune response and the nanoparticle induce a cell mediated immune response.

Another embodiment of the invention provides a vaccine composition, wherein the antigen is derived from an organism selected from a group consisting of rotavirus, paramyxovirus, mumps virus, rubella virus, polio virus, hepatitis virus, herpes viruses, human immunodeficiency virus, *Haemophilus influenza, Clostridium tetani, Corynebacterium diphtheria,* and *Neisseria gonorrhea.*

Another embodiment of the invention provides a vaccine composition, wherein, said antigen is selected from a group of Hepatitis B surface antigen, tetanus toxoid and Diphtheria toxin.

Another embodiment of the invention provides a vaccine composition, wherein the antigen is selected from a group consisting of proteins, polysaccharides, glycoproteins, glycolipids, nucleic acid and combinations thereof.

The vaccine composition of the present invention, as defined herein above, is characterized in that:
the size of said nanoparticle ranges from 200 -600 nm;
the size of said microparticle ranges from 2-8 µm; and
the polymeric particles are of a biodegradable polymer.

Said biodegradable polymer may be selected from a group of poly (lactide-co-glycolide), polylactide or polyglycolide.

Preferably, said polymeric particle is polylactide (PLA) polymer.

Another embodiment of the invention provides a vaccine composition, as defined herein above, for use for treating diseases selected from a group consisting of malaria, tuberculosis, and human immunodeficiency virus infection.

One embodiment of the present invention provides a vaccine composition, as defined herein above, for use for eliciting a combined humoral (bioneutralizing antibody) and cellular response or cell-mediated immune response (CTL, T helper cell) in a subject, the composition comprising an antigen loaded in a polymeric composition of nanoparticles and microparticles, wherein the polymeric particles are of a polymer of poly(lactide-co-glycolide), polylactide or polyglycolide.

The microparticle and nanoparticle polymer may be loaded with the same antigen promoting both humoral and cell mediated responses in a balanced manner or in a manner as desired.

The vaccine loaded polymer particles, as defined herein above, are for use in the treatment of diseases or clinical manifestations that require both bioneutralizing antibodies and T cell mediated responses, such as malaria, tuberculosis, cancer and human immunodeficiency virus infection.

One embodiment of the present invention provides biodegradable polylactide (PLA) polymer particles as vaccine delivery systems to elicit either or both humoral and cell mediated immune responses from the same antigen. The difference in size and release pattern of polymer entrapped antigen(s) are known to influence the way the antigen(s) is/are presented by antigen presenting cells (APCs) to activate naïve CD4⁺T cells which differentiate upon activation into either Th1 or Th2 cells resulting in either cell mediated or humoral immune responses respectively. Th1 cells are associated with IFN-y, IL-12 and TNF-α whereas Th2 cells typically produce IL-4, IL-5, IL-10 and IL-13. The present invention provides a combined antibody response and T cell mediated response in an animal model from different sized PLA polymer particles with entrapped HBsAg.

One embodiment of the present invention provides a size based design of vaccine delivery system for modulation of immune response from the same antigen. The particulate antigen acts as an effective immunogen for therapeutic, prophylactic and vaccination purposes with eliciting both humoral and cell mediated responses in a balanced manner. Generation of different magnitudes and kinetics of immune response from a mixture of microparticles and nanoparticles and without adjuvants like alum suggests that particle size is an important parameter influencing the immune response.

The exact amount of such compositions required will vary from subject to subject, depending on the species, age, weight and general condition of the subject, the severity of the disease, infection or condition that is being treated or prevented, the particular compound used, its mode of administration, and the like. Thus, it is not possible to specify an exact amount. However, an appropriate amount may be determined by one of ordinary skill in the art using only routine experimentation given the teachings herein.

One embodiment of the present invention provides a single shot vaccine delivery systems, as defined herein above, that can be delivered parenterally, intrapertonially, orally, topically, transdermally, intradermally, rectally, vaginally or nasally.

The preparation of polymer particles and characterization of particle size and surface morphology are provided in Example 1 and Example 2.

Immunogenicity evaluation of PLA particles containing HBsAg. Immunization of rats with HBsAg encapsulated in micro-particles and nanoparticles of PLA with or without alum is shown is Example 3. Immunization with different sized HBsAg entrapped particles result in varied antibody response (Figure. 1). HBsAg entrapped microparticles (2-8 µm) elicit better antibody titer (peak titer value 1803±150 mIU/mL) than nanoparticles (peak titer value 1010±191 mIU/mL). Admixture of microparticles and alum improve the antibody titers considerably. Immunization with microparticles and alum (MP+Alum) elicit antibody titers (peak titer value 4941+100 mIU/mL) better than from alum adsorbed HBsAg (peak titer value 3999±150 mIU/mL). The data is provided in Figure 1 and table 1.

Estimation of HBsAg antibody titer by performing ELISA. The details for ELISA are provided in Example 4.

Example 5 shows a Tell proliferation and cytokine assay the microparticles which give rise to higher antibody response promote IL-4 secretion; whereas nanoparticles favor IFN-γ secretion. This data is provided in Figure 5 and table 2, 3 and 4. This is in concurrence with the concept that microparticles promote humoral response where as nanoparticles promote cellular response. The nanoparticles escape into the cell cytoplasm and deliver the antigen in the cytosol which gets acted upon by MHC class I presentation machinery thereby promoting more of Th1 type response where as immunization with microparticles results in antigen presentation via the classical MHC II presentation on the APCs and thus promote Th2 response. Analysis of surface MHC levels on APCs *in vitro* further supports this fact. In case of macrophages treated with nanoparticles (200-600 nm), surface MHC class I levels are greatly upregulated at 24 hours. But in case of macrophages treated with microparticles (2-8 µm and beyond), MHC class II levels are significantly upregulated than MHC I as shown in Figure 7.

It can be shown that microparticles promote humoral response where as nanoparticles promote cellular response. Accordingly, microparticles promote Th2 response and nanoparticles promote TH1 response. This is a surprising result of the present invention as this result is in contradiction to the earlier teachings that disclose that microparticles promote Th1 response and nanoparticles promote TH2 response.

Example 6 shows *in vitro* and *ex vivo* analysis such as Phagocytic uptake, Fluorescence quenching , Confocal laser scanning microscopy (CLSM) and flow cytometric analysis of fluorescent particles.

Cellular interactions of different sized PLA particles with macrophages are analyzed to visualize particle uptake by macrophages, cells are incubated with different sized PLA particles for various time points both in vitro and ex vivo as shown in Example 6. Fluorescence microscopy shows that most of the nanoparticles were localized intracellularly and maximum uptake is observed between 6-24 hours. It is also observed that nanoparticles get continuously endocytosed and exocytosed from the cells (Figure 2A-D). The most interesting observation was that PLA microparticles of size 2-8 µm and above were found to be attached to the surface of APCs for a considerable period of time (Figure 2E-H). Intracellular accumulation of nanoparticles was associated with 2-3 times enlargement of cell size (Figure 2I) where as association of microparticles did not alter the cell size (Figure 2J). It has been documented that size and surface chemistry of particles do influence particle uptake and in particular, hydrophobic polymer particles have the tendency to adhere to the cell surface. Higher sized particles (20-50 µm) which have been reported previously to elicit antibody titer upon immunization were also found to be attached to the cell surface (Figure 2K).

Attachment of PLA microparticles on cell surface of macrophages was analyzed to confirm that fluorescence emitted from the microparticles is indeed extracellular and hence the microparticles are cell surface attached, 0.1 % (w/v) crystal violet or 0.4 % (w/v) trypan blue is used to quench the extracellular fluorescence. Under such conditions only intracellular fluorescence signal would be emitted and captured and any extracellular signal would get nullified by the quenching dye. FITC-BSA entrapped nanoparticles are efficiently up taken by macrophages and appeared green under FITC filter (Figure 3, A-C). In case of FITC-BSA entrapped microparticles, fluorescence is quenched by the dye confirming that they are cell surface associated (Figure 3, D-F). Also, in case of FITC-BSA entrapped microparticles, green fluorescence is only seen intracellularly and not inside the particles (Figure 3, E and F). This can be explained by the fact that microparticles remain cell surface adherent non-specifically and constantly release the fluorescent antigen (BSA) into the cell. This results in intracellular accumulation of antigen as reflected in higher levels of fluorescence. In such cases, the positions on the cell surface where the microparticles are attached do not show any fluorescence instead certain specific areas within the cell appear green under the FITC filter. The uptake of particles is quantified as cell-associated fluorescence intensity by cytometric analysis using a BD-LSR flow cytometer (FACS). The fluorescence quenching for 6-coumarin entrapped PLA microparticles both in absence and presence of a phagocytic inhibitor cytochalasin D. It is observed that very low percentage of cells show fluorescence at all time points upon quenching. Since the extracellular fluorescence is quenched by trypan blue (≧ 85 %), it indicates that these microparticles are extracellular and cell surface attached. Similar results are observed in the presence of cytochalasin D (20 µg /1 x 10⁶ cells). Percentage of fluorescence labeled cells drastically reduced from as high as 25.4 % to 2.2 % at 24 hours, and 15.9 % to 0.4 % at 48 hours when quenched with trypan blue. Since cytochalasin D, only prevents phagocytosis but not cell surface attachment per se, these results prove that microparticles (2-8 µm) are indeed attached to the cell surface. Similar observations are found for bigger sized (>10 µm) particles. However, 6-coumarin entrapped PLA nanoparticles are efficiently taken up by macrophages in a time bound manner indicated by an increase in fluorescence intensity which saturates and decreases at later time points in the presence of trypan blue (Figure 4B). Even after quenching, fluorescence of 6-coumarin entrapped nanoparticle increase from 1.8 % (1 hour) to 36.6 % (at 24 hours) but decrease at later time points (18.4 % at 48 hours). This reduction in nanoparticle uptake at higher time point may be attributed to the exocytosed nanoparticles which are quenched by the dye after a long period of incubation. However, in the presence of cytochalasin D, a drastic decrease in percentage fluorescence of nanoparticles is observed (Figure 4B, lower panel). Most of the fluorescence associated with nanoparticles is quenched and is around 1-2 % at all the time points. Cytochalasin D prevents the phagocytosis of nanoparticles and hence the fluorescence from extracellular nanoparticles is quenched.

Analysis of *in vitro* localization of different sized PLA particles in macrophages by CLSM. Intracellular localization of nanoparticles and surface attachment of microparticles is confirmed by CLSM. In case of macrophages incubated with fluorescent nanoparticles, a number of nanoparticles are found present in different optical sections (z-sections) of the same cell. This implies that nanoparticles are indeed phagocytosed by the cell and localized at different locations (Figure 4A and B). In case of macrophages incubated with microparticles, the same microparticle is visible in all optical sections. Microparticles appear in focus in the initial sections much before the cell actually can be focused with respect to the optical sectioning done for a particular cell sample indicating that microparticles are cell surface associated and not internalized (Figure. 4C and D). For analyzing intracellular localization of particles, cells are later incubated with organelle-specific contrasting fluorescent dye, 50 nM Lyso Tracker^{™} Red (Molecular Probes) for 30 minutes at 37°C and washed three times with sterile 50 mM phosphate-buffered saline (PBS). The endo-lysosomes appear red in color when labelled with 50 nM Lyso Tracker^{™} Red and visualized under RITC filter. A series of z-sections are performed using CLSM following which the co-localization of the nanoparticles within the endo-lysosomal compartments at different sections of the cells can be visualized under both FITC and RITC filters. The co-localization shows yellow fluorescence in the overlay between green fluorescent nanoparticles and red fluorescent vesicles at various time points. No such co-localization of the fluorescent microparticles is observed within the labelled cells though they are seen to be attached to the surface of the cells. The cellular attachment of microparticles is evident even after vigorous washing of the particles treated cells with 50 mm PBS.

Analysis of cytokine secretion pattern, antibody isotyping, and surface MHC levels on interaction of different sized antigen loaded PLA particles with macrophages. As the modulation of the immune response is regulated by cytokines, their secretion pattern is analyzed from T cells, isolated from mice immunized with different sized PLA particles entrapping HBsAg. Cytokine levels are quantitated from the culture supernatants of T-cell proliferation assay (Figure 5A). It is observed that nanoparticles favor IFN-γ secretion (Figure 5B) much higher (4-5 times) in comparison to microparticles. Microparticles, on the other hand, favor IL-4 secretion which is better than that from nanoparticles (Figure. 5C). This supports the observation that nanoparticles do enter the cells and somehow escape to the cell cytoplasm to promote Th1 response. Microparticles remain outside the cell and promote Th2 response. This is further confirmed from the antibody isotyping in rats immunized with different sized HBsAg encapsulated PLA particles over a period of one month. Groups immunized with microparticles show higher IgG1/IgG2a ratio than those immunized with nanoparticles (Figure 6). IgG1 is an indicator of Th2 type response whereas IgG2a favors Th1 type response. Alum is a known Th2 favoring adjuvant thus immunization with only alum favored highest IgG1/IgG2a ratio. Immunization with admixture of microparticles and alum favors three times more IgG1/IgG2a ratio than that observed with nanoparticle and alum immunization. Nanoparticles alone result in lowest IgG1/IgG2a ratio suggesting the preferential elicitation of cellular immune response. Improvement in antibody titer as well as IgG1/IgG2a ratio with admixture of alum and nanoparticles are not as significant as it is observed with microparticle arid alum based immunization (Figure 1 and Figure 6). Different sized PLA particles entrapping the same antigen (HBsAg) thus elicit immune response of varied strength and quality. Modulation of immune response from different sized HBsAg particles is further characterized by analysis of surface MHC levels on macrophages *in vitro* (Figure 7). HBsAg microparticles (2-8 µm, 20 µm and 50 µm) upregulate MHC class II molecules significantly and correlate with high IL-4 secretion and higher antibody titers. Polylactide nanoparticles (size range 200 nm-600 nm) upregulate higher levels of surface MHC class I molecules in comparison to MHC class II molecules. This analysis is done in a size specific manner rather than antigen specific manner and it further supports the observations obtained from cytokine analysis and antibody isotyping using different sized HBsAg particles. Therefore the present disclosure shows unexpected result of eliciting Th2 and TH1 response by microparticles and nanoparticles respectively. This surprising finding of the present invention is exemplified by using a combination of microparticles of size 2-8 µm and nanoparticles of size 200 nm-600 nm.

Intracellular protein analysis by western blot of cell lysates of macrophages treated with different sized HBsAg entrapped PLA microparticles. Shown is an increased intracellular concentration of soluble HBsAg with time (Figure 8A, 8B and 8C). The detail of the Western blot protocol followed is described in Example 6. Even after 24 hours of incubation with microparticles, macrophages show increased intracellular levels of HBsAg in comparison to nanoparticles. In case of nanoparticles, level of HBsAg detected is prominent in the initial time point (Figure 8A) but was more or less constant at later time points (Figure 8B, 8C). These results further corroborate with higher levels of FITC-BSA accumulation inside APCs released from microparticles in comparison to nanoparticles at similar time points (Figure 3E and F). This observation offered a clue as to how the interaction between the APCs and antigen loaded PLA microparticles elicits higher antibody response.

The present experimental data clarify the ambiguity relating to whether the vaccine loaded hydrophobic PLA microparticles elicit antibody response without being phagocytosed by the APCs and how different sized antigen loaded polymer particles modulate immune response. In the earlier studies it has been reported that as the particle size decreases from micrometer range to nanometer range, antibody titer decreases. Similar differences in antibody titers are observed while immunization with different sized HBsAg particles. Immunization with microparticles result in long lasting antibody titers in comparison to nanoparticles from single point immunization. Large sized particles (larger than APCs) do not get phagocytosed but still elicit antibody response from single point immunization. This suggests that uptake of the particulate antigen through phagocytosis and subsequent processing through classical MHC **II** presentation pathway may not be operating for such immunization modalities. Even though extensive research reports on phagocytosis of nanoparticles are available, uptake of different sized particles (micron sized) have never been correlated with antibody response.

The role of particle size in antigen presentation leading to generation of antibody response is assessed by carrying out cellular uptake with different sized fluorescent PLA particles. Nanoparticle endocytosis and its intracellular localization are conclusively proved by CLSM. Fluorescence quenching in presence and absence of cytochalasin D indicated that nanoparticles are uptaken by APCs through phagocytosis but microparticles are surface attached. Since the localization using CLSM show that nanoparticles are internalized and located in different compartments within the cell, it is safe to assume that most of the entrapped antigen is released into different intracellular compartments. Continuous endocytosis and exocytosis of nanoparticles from APCs probably results in low concentration of intracellular antigen with time as observed in western blots. Immunization with antigen loaded nanoparticles is associated with secretion of IFN-γ and upregulation of MHC class I molecules. Nanoparticle thus delivers antigen intracellularly and promotes more of cellular response. As both arms of immune system are complementary to each other this results in lower IgG antibody titers with nanoparticle based immunization in comparison to microparticle based immunization. However, to our surprise, micro particles (2-8 µm size) that elicit optimal antibody titer upon immunization are not taken up by the APCs. More over, they are found to be attached to the cell surface for a considerable period of time non-specifically as observed by confocal as well as flow cytometric analysis. Particle surface chemistry and size play an important role in deciding the cellular localization of polymer particles. It has been shown that hydrophobic particles of size around 1 µm remain associated with early endosome and thus never get phagocytosed to lysosome. The hydrophobic nature of PLA microparticles helps in stronger attachments to cell membrane and large size (>1 µm) prevents the internalization of PLA microparticles. This results in cell surface attachment of PLA microparticles for a considerable period of time from where the entrapped antigen is released continuously into the cell. This is confirmed by the presence of higher levels of intracellular HBsAg from western blot analysis. Microparticles thus deliver the antigen slowly but persistently which results in improved antibody response in comparison to nanoparticle based immunization. However, it must be noted that the compartmentalization of the released antigen is not assessed per say but these results stress the point that there is continuous release of antigen from the microparticles into the cells for a longer period of time. This is further supported by the observation that similar sized particles having high load of antigen elicits higher antibody response from PLA particle based immunization. Since these particles are not taken up by APCs, surface attachment and continuous release of antigen from microparticles at high concentration influences the immunogenicity of polymer entrapped antigen. Higher secretion of IL-4 and upregulation of MHC II molecules induced by microparticles preferentially favor the processing and presentation of antigen through MHC class II pathway resulting in dominant humoral response. An optimal size of microparticles thus improves antibody response from single point immunization using polylactide particles. As the particle size increases beyond 2-8 µm, the available surface area of the antigen loaded particles for attachment to APC decreases resulting in low antibody titers.

These results show that uptake of microparticles is not an absolute requirement for efficient antibody generation from PLA particle based immunization. Uptake of a pathogen or antigen normally takes either of three pathways. Firstly, APCs are capable to engulf particles or microorganisms non-specifically. Secondly, phagocytes are equipped with several cell-surface receptors that recognize pathogen surfaces for preceptor-mediated endocytosis. Thirdly, phagocytic cells can take up soluble substances in pinocytic vacuoles by a process called macropinocytosis. For presentation of extra cellular antigen, it needs presentation and processing along with MHC II binding. The extra cellular antigen can bind to MHC II molecules present in the plasma membrane, MHC II molecules generated in early phagosomes or those generated by late endocytic pathways. For antibody generation, it is essential that the peptide fragment of the antigen binds to the MHC II molecule. This can happen either to membrane bound, recycled or nascent MHC II molecules. Even though membrane bound MHC constitute around 30 % of the total molecules present in the cell for antigen presentation, these molecules are sufficient for generation of antibody response from polymer entrapped antigen. As disclosed by previous publications particulate involving latex beads where the antigen is covalently linked, PLA particles continuously release the antigen. Thus, in case of micron sized polymer particle, there is continuous release of antigen from the particles attached to the plasma membrane of the APCs for a considerable period of time. Plasma membrane has the capacity to process and present antigen and this may be the dominant mechanism for antigen presentation following immunization with PLA entrapped microparticles.

These results also show that these higher sized polymer particles remain attached to the APCs for a considerable time and help in presentation of antigen via MHC II pathway which was conclusively proved. It is possible that particle entrapped antigen is directly presented to B cells through antibody like receptor on its surface. Continuous release of antigen in the vicinity of APCs has been reported to be enough for presentation through membrane bound MHC II molecules. High concentration of soluble antigen near the extra cellular space of the APCs can lead to direct loading of the antigen onto MHC II molecule for presentation or can be transported by fluid phase macropinocytosis for further processing and presentation. Apart from macrophages, DCs and B cells are other APCs which may be involved in presentation of polymer entrapped antigen for improved immune response. DCs are considered to be the most important APCs; however contradictory reports exist about the ability of PLGA polymer particles to cause maturation of DCs into antigen presenting cells. These polymer particles do not possess any pathogen associated molecular patterns (PAMP) to activate DCs. However, DCs help in prolonging presentation of antigen from polymer entrapped particles. Recent studies suggest that the improved immunogenicity of the polymer entrapped antigen is mostly because of passive targeting of entrapped antigen into APCs.

These results further show that immunogenic potential of different sized particles by analysis of interactions between antigen loaded polymer particles with macrophages. Based on the *in vitro* and *ex vivo* uptake example in murine macrophage cell line J774A. 1 and murine peritoneal exudate cells; and particle size based immunization *in vivo* it is concluded that: (i) Hydrophobic PLA microparticles (2-8 µm size) preferentially attach to the cell surface of APCs (macrophages) and continuously release the entrapped antigen resulting in higher antibody response than nanoparticles (<1 µm). (ii) PLA nanoparticles (<1 µm) are efficiently phagocytosed by APCs (macrophages) and promote cell mediated immune response thus it elicits lower antibody titers upon immunization. (iii) Intracellular uptake of antigen loaded PLA microparticles is not necessary for improved antibody response from single point immunization. (iv) Polymer particle entrapped antigen are probably presented and processed in multiple ways for improved immune response, however passive targeting remains one of the major advantages of such system. (v) Size of polymer particle based antigen delivery system thus provides an exciting possibility for modulation of immune response via differential interactions with macrophages.

These results show that, for antibody response, hydrophobic PLA particles are better than hydrophilic polymer particles and microparticles (2-8 µm size) entrapping tetanus toxoid elicit optimal antibody titers. Immunization with higher sized polymeric particles (> 10 µm and < 50 µm) as well as nanoparticles also elicit comparable antibody titers but lower than that observed for microparticles of 2-8 µm size.

The results finally show that intracellular uptake of PLA microparticles per se is not an absolute requirement for effective and improved antibody response from particle based immunization. Instead, the continuous release of high concentration of antigen from PLA microparticles attached to the surface of APCs for a considerable period of time results in improved antibody response. Size of the polymeric particulate antigen delivery system and its subsequent interaction with macrophages is crucial for modulating immune response from single point immunization.

From these results it is suggested that it is possible to modulate the type of immune response from the same antigen varying concentration of the different size of the polymer particles as per requirement of humoral and cell mediated response.

The invention is further illustrated by the following examples, which are not to be construed in any way as imposing limitations upon the claimed subject matter.

### EXAMPLES

PLA (45 kDa) was purchased from Birmingham Polymer Inc. USA. Recombinant HBsAg was from Shantha Biotechnics Pvt. Ltd, Hyderabad, India. Alum (2 % w/v Al-hydrogel) was from Superfos Biosector, Denmark. Fluorescein isothiocyanate conjugated bovine serum albumin (FITC-BSA) [A-9771], rat serum albumin (RSA) [A-6272], cytochalasin D [C-8273] and polyvinyl alcohol, MW 30,000 (PVA) were from Sigma Chemicals, USA. 6-coumarin (8037L) was from Polysciences, Warrington, PA. Dulbecco's modified Eagle's medium (DMEM), fetal calf serum (FCS) and Roswell Park Memorial Institute (RPMI-1640) medium were from Biological Industries, Israel. Micro BCA protein assay kit was from Pierce, USA. Seratec EIA hepatitis B antibody kit was from Seratec Inc. Germany. Goat anti-HBsAg antibody was from Novus Biologicals, Inc. USA and HRP conjugated anti-Goat IgG antibody was from Bio-Rad, USA. HRP conjugated anti-rat IgG1 and anti-rat IgG2a antibodies were from AbD Serotec, USA. Cell Tracker^{™} Red and Lyso Tracker^{™} Red were from Molecular Probes, Eugene, Oregon, USA. Mouse IL-4 ready-set go ELISA kit, PE labeled anti-mouse CD 11b and PE labeled anti-mouse MHC class I, H-2K^{d}/ H-2D^{d} antibodies were from eBiosciences, Inc. Mouse IFN-γ BD OptEIA^{™} ELISA set and FITC labeled anti-mouse MHC class II (I-A/ I-E) were from BD Biosciences, San Diego, CA. Brewer Thioglycollate medium (M019) was from HiMedia Lab., India. Glass fibre filter, printed filtermat A, was from Wallac, Finland. Western blot reagents and hyper film were from Amersham Biosciences, USA.

### EXAMPLE 1

### Preparation of polymer particles

Polylactide polymer particles were prepared using w/o/w double emulsion solvent evaporation method (Katare YK, Muthukumaran T, Panda AK. Influence of particle size, antigen load, dose and additional adjuvant on the immune response from antigen loaded PLA microparticles. Int J Pharm 2005;301:149-60.). Briefly, primary emulsion between internal aqueous phase (IAP) containing HBsAg (10 mg/ml) and organic phase (OP) (50 mg/mL PLA solution in dichloromethane) was prepared by sonication (20 watts, 40 % duty cycle, 20 cycles) (Branson Sonifier 450, USA). Resulting primary emulsion was added drop wise to external aqueous phase (EAP) containing 1 % (w/v) PVA and 10 % sucrose (w/v) solution in MQ water and homogenized (10,000 rpm for 10 minutes) using a homogenizer (Virtis, Cyclone I.Q., USA) for microparticle and macroparticle preparation; and sonicated (20 watts, 40 % duty cycle, 20 cycles) for nanoparticle preparation. The resulting particles were collected by centrifugation (15,000 rpm, 20 minutes), and lyophilized to get free flowing powder. For the preparation of 6-coumarin fluorescent particles, size ranging from < 1µm to > 25 µm, 50 µL of 6-coumarin dye (1 mg/mL in dichloromethane) was also added to OP during primary emulsion step. Similarly for the preparation of FITC conjugated BSA particles, FITC-BSA (10 mg/mL) was taken in IAP as the antigen. Different sized fluorescent and HBsAg entrapped PLA particles were prepared by varying energy input and OP to EAP volume ratio (Katare YK, Muthukumaran T, Panda AK. Influence of particle size, antigen load, dose and additional adjuvant on the immune response from antigen loaded PLA microparticles. Int J Pharm 2005;301:149-60.).

### EXAMPLE 2

### Characterization of particle size and surface morphology

Size distribution of the particles was determined using Malvern mastersizer 2000 particle size analyzer (Malvern, UK). Surface morphology was analyzed by using a scanning electron microscope (SEM) - JEOL (JSM 6100, Tokyo, Japan) - after coating the particle surface with gold-palladium over an aluminium stub; and also by a transmission electron microscope (TEM) - CM 10, Philips, Holland - after coating the particles with 1 % uranyl acetate over a copper grid (Polysciences, Warrington, PA). TEM images were obtained by using digital imaging software - AMT image capture engine (version 5.42.391). To measure the protein content of particles, accurately weighed particles were dissolved in acetonitrile to solubilize the polymer while precipitating the encapsulated protein. Precipitated protein was dissolved in 1 % SDS solution and estimated using micro BCA assay. Protein extracted from particles was resolved in SDS-PAGE gel and the amount of protein in the mixture was determined by densitometry scanning of gel picture. Protein loading was calculated as the percent weight of protein per unit weight of polymer.

### EXAMPLE 3

### Immunogenicity evaluation of PLA particles containing HBsAg

Immunogenicity of PLA particles containing HBsAg was evaluated in Wistar rats (eight female out bred Wistar rats per group). Animals were maintained according to the guidelines established by the Institute Animal Ethics Committee (IAEC) of the National Institute of Immunology, New Delhi. Required dose of particles were weighed and suspended in saline just before immunization. Immunization of admixture of particles and alum were carried out by adding 25 µL of alum (Aluminium hydroxide gel, 2 % w/v) to the required dose of polymer particles per animal. Single dose of alum adsorbed antigen (1 µg HBsAg) was used as control. Rats were immunized intramuscularly with 1 µg of HBsAg encapsulated in micro- and nano-sized PLA particles with or without alum. Animals were bled at different time intervals through retro-orbital plexus and serum antibody titers were determined by ELISA.

### EXAMPLE 4

### Estimation of HBsAg Antibody titer by ELISA

For the estimation of anti-HBsAg IgG antibody titers in serum samples, SERATEC (GmbH, Germany) HBsAg EIA kit was used. Briefly, 50 µL of each serum sample dilution was added in duplicates in a 96-well microtiter plate pre-coated with HBsAg. Antibody standards, ranging from 10 to 150 mIU/mL, were loaded in duplicates. Immediately, 50 µL conjugate (HRP-labeled HBsAg) was added to each well and microplate was incubated for 30 minutes at 37° C. Microplate was then washed five times with wash buffer. This was followed by addition of 50 µL of chromogen A (containing hydrogen peroxide) and 50 µL of chromogen B (containing TMB (tetra methyl benzidine)) in each well and mixed properly. Microplate was again incubated at 37° C for 15 minutes. Reaction was stopped by adding 5N H₂SO₄ (50 µL/well) and absorbance was measured at 450 nm. Antibody titers were defined as the serum anti-HBsAg IgG antibody levels in mIU/mL. Anti-HBsAg IgG antibody titers were estimated in duplicates and their concentrations (mIU/mL) were determined as geometric mean (n = 8). Analysis of, immune response modulation was performed by qualitative estimation of anti- rat IgG1 and anti-rat IgG2a antibody titers in the serum samples of rats immunized with different sized particles entrapping 1µg HBsAg. Briefly, 96-well high binding flat-bottom EIA/RIA plates (Nunc) were coated with 200 ng HBsAg/100µl per well for I ½ hours at 37° C. Plates were washed once with wash buffer (PBS-T) and then blocked using blocking solution (1% BSA in PBST) for 1 ½ hours at 37° C. After washing, serum samples were loaded in duplicates and incubated again for 1 ½ hours at 37° C. Plates were then extensively washed four times with wash buffer. This was followed with addition of secondary anti-rat IgG1 *HRP or IgG2a*HRP conjugate antibody and incubated for 1 hour at 37° C. Finally, 100 µL of O-phenyl diamine (OPD) dissolved in citrate-phosphate buffer (pH 4.5) along with H₂O₂ was added to each well and incubated for 20 minutes at room temperature. Reaction was then stopped using 50 µl per well of 2N H₂SO₄ as the stop solution and absorbance was read at 492 nm using an ELISA reader. Ratio between IgG1 and IgG2a absorbance values for different size particle were analyzed.

### EXAMPLE 5

### T cell proliferation and cytokine assay

For T-cell proliferation assay, pathogen-free 6-8 weeks old inbred male BALB/c mice (n = 3) were subcutaneously immunized with either HBsAg encapsulated nanoparticles; microparticles; or alum adsorbed soluble HBsAg containing 10 µg HBsAg suspended in 125 µL saline per animal. Non-immunized group of animals was taken as control. One week after immunization, T cells were isolated from popliteal and inguinal lymph nodes of the euthanized animals. A single-cell suspension was prepared by smearing cell aggregates/lymph nodes between two sterile frosted slides in sterile RPMI medium. T cells (0.3 x 10⁶ cells per well) were incubated in triplicates with serially double diluted soluble HBsAg as the recall antigen ranging from 10 µg/100 µL downwards at 37° C and 5 % CO₂. No antigen was added for the control group. After 72 hours post incubation, 50 µL culture supernatant was removed from each well for cytokine assay and 0.5 µCi ³H-thymidine/ 50 µL RPMI medium was added to each well and incubated for a further 14-16 hours after which the plates were harvested on to a glass fibre filter, printed filtermat A, using a Strakon cell harvester and then incorporated radioactivity was measured in a liquid scintillation counter (Wallac 1205 Betaplate counter).

Cell culture supernatants collected from T-cell proliferation assay were analyzed for mouse IL-4 and/or IFN-γ using eBioscience and BD ELISA kits respectively. Briefly, 96-well microtiter plates were coated with recombinant capture antibody at a dilution of 1/250 (50 µL/well), anti-mouse IL-4 and anti-mouse IFN-γ for quantitative detection of mouse IL-4 and IFN-γ respectively, and incubated at 4° C overnight. Plates were then washed two times with PBS-T and once with PBS and then blocked using blocking buffer (200 µL/ well) at 37° C for 1-2 hours. Sets of standard serial dilution of recombinant mouse IL-4 (1 µg/mL) and IFN-γ (90 ng/mL) were prepared and added to the corresponding microplates along with the pooled test samples in individual wells (50 µL/well) and incubated for 1-2 hours. The mouse IL-4 standards ranged from a top standard of 500 pg/mL to serially diluted bottom standard of 15 pg/mL whereas the mouse IFN-γ standards ranged from 1000 pg/mL to 31 pg/mL. All dilutions were made using RPMI-1640 cell culture medium. Detection was performed using 50 µL/well of biotinylated anti-mouse IL-4 and IFN-γ antibodies respectively at 1/250 dilution followed by addition of avidin-HRP enzyme at a dilution of 1/250. TMB was used as the substrate. After 15 minutes incubation at room temperature, reaction was stopped using 2N H₂SO₄ and absorbance was read at 450 nm.

### EXAMPLE 6

### In vitro and ex vivo Analysis

### Phagocytic uptake

*In vitro* and *ex vivo* phagocytic uptake and trafficking analysis were carried out using 6-coumarin and FITC-BSA encapsulated polymer particles in murine macrophage cell line J774A.1 and murine peritoneal exudate cells. For *in vitro* analysis, 25 µL (1 mg/mL) of different sized fluorescent particles- 400 nm, 2-8 µm or > 10 µm - was added to 0.5 x 10⁶ cells/ 3 mL of DMEM medium supplemented with 10 % FCS, 100 U/mL penicillin, 100 µg/mL streptomycin and 0.25 µg/mL amphotericin B plated in sterile standard tissue culture grade 6-well plates (Falcon, Becton Dickinson, Franklin Lakes, NJ) and incubated at 37° C, 5 % CO₂ for various time points. Cells were washed three times with sterile 50 mM PBS and observed using a fluorescent microscope. For *ex vivo* analysis, 6-8 weeks old male BALB/c mice were each administered with 1 mL of 4 % thioglycollate solution intraperitoneally. After three days, mice were euthanized by cervical dislocation and peritoneal macrophages were collected by flushing out the peritoneal cavity with 10 mL of ice cold RPMI-1640 medium. Cell suspension was centrifuged at 1,200 rpm for 5 minutes at 4°C and resuspended in RPMI-1640 medium supplemented with 10 % FCS, 100 U/mL penicillin, 100 µg/mL streptomycin and 0.25 µg/mL amphotericin B. Only adherent cells, 95 % of which constitute macrophages, were treated with fluorescent particles and processed as described above.

### Fluorescence quenching

The presence of fluorescent particles in macrophages were tracked using a Nikon ECLIPSE TE 2000-U fluorescent microscope fitted with a digital camera (1200 DXM) and the images were obtained using Image Pro-Plus 5.1 software program. Briefly, 6-well plates containing macrophages were treated with different sized FITC-BSA based fluorescent PLA particles for different time points and washed with sterile PBS solution. Quenching of extra cellular fluorescence was achieved using 0.1 % (w/v) crystal violet or 0.4 % (w/v) trypan blue solution [Rejman J, Oberle V, Zuhorn IS, Hoekstra D. Size-dependent internalization of particles via the pathways of clathrin- and caveolae-mediated endocytosis. Biochem J 2004;377:159-69.; Hed J, Hallden G, Johansson SG, Larsson P. The use of fluorescence quenching in flow cytofluorometry to measure the attachment and ingestion phases in phagocytosis in peripheral blood without prior cell separation. J Immunol Methods 1987;101:119-25]. Images were captured using 40X objective lens under FITC filter and also under bright light in monochrome mode. Fluorescence mode images and monochrome mode images were then merged using Image Pro-Plus software.

### Confocal laser scanning microscopy (CLSM)

CLSM images were obtained by simultaneous scanning of contrasting double- labeled specimens using a Zeiss Confocal LSM510 microscope equipped with Argon-Krypton laser (Carl Zeiss Micro imaging, Inc., NY, USA). Macrophages were grown on cover slips inside sterile 6-well tissue culture grade plates and incubated with fluorescent nanoparticles or microparticles at 37° C, 5 % CO₂ for various time points; washed with 50 mM sterile PBS and then labeled with 50 nM Cell Tracker^{™} Red or 50 nM Lyso Tracker^{™} Red at 37° C for 30 minutes and again washed three times with 50 mM sterile PBS. The cover slip was then placed on the stage of confocal microscope. A representative cell was selected at random and a series of optical sections (z-sections) were taken in dual filter mode. Images captured in RITC, FITC and dual mode were overlaid to determine localization and co-localization of fluorescent particles.

### Flow cytometric analysis of fluorescent particles

Cytometric acquisition of fluorescent particle uptake analysis was performed using a BD-LSR flow cytometer (BD Biosciences, San Jose, USA) and Cell Quest program. The data was analyzed using WinMDI 2.8 (Joseph Trotter, Scripps Institute, La Jolla, CA). Macrophage cell suspension (0.5 x 10⁶ cells) pre-incubated with fluorescent nanoparticles (25 µg) or microparticles (25 µg) for various time points (at 37° C, 5 % CO₂) was analyzed for presence or absence of internalized fluorescent particles. Extra-cellular fluorescence was quenched using an appropriate dye (0.1 % w/v crystal violet or 0.4 % w/v trypan blue). Particle uptake inhibition analysis was done using cytochalasin D. For this, macrophages were pre-incubated with cytochalasin D (20 µg /1 x 10⁶ cells) 30 minutes before the start of the experiment and continued till the end.

Effect of particle size was also analyzed in terms of surface MHC levels on APCs - murine macrophage cell line J774A.1 *- in vitro.* Briefly, 0.5 x 10⁶ cells were cultured in complete DMEM medium and incubated with different sized particles encapsulating 1 µg HBsAg for different time points. After washing with sterile PBS, cells were incubated with PE anti-mouse MHC class I and FITC anti-mouse MHC class II (I-A/I-E) on ice for 30 minutes. Untreated cells were used as control. Surface MHC class I and MHC class II levels on APCs were acquired using a BD-LSR flow cytometer and data was analyzed using WinMDI 2.8.

### Intracellular protein analysis by Western Blot

Intracellular protein released from particles was analyzed after incubation of murine macrophage cell line J774A.1 with different sized HBsAg loaded PLA particles *in vitro.* 0.5 x 10⁶ cells per well were cultured in sterile 6-well plates at 37° C, 5 % CO₂. Different sized particles containing 1 µg HBsAg were added to individual wells at around 70 % confluence and incubated for three different time points- I hour, 6 hours, and 24 hours. After each incubations, cells were washed with sterile PBS and pelleted at 1200 rpm for 5 minutes at room temperature. Cell lysates were analyzed for presence of HBsAg by western blotting using chemiluminescence method.

### EXAMPLE 7

### Statistical analysis

For statistical analysis, anti-TT antibody titers were defined as µg of antibody per ml of the sera employing affinity purified antibody as a reference in ELISA. Antibody titers of individual animal (n = 8) were estimated in duplicates and their concentrations (µg/ml) were determined as geometric mean. Anti-HBsAg antibody titers were estimated in duplicates and their concentrations (mIU/ml) were also determined as geometric mean (n = 6). Cytokine concentrations in the cell culture supernatants were determined from the standard curve (regression coefficient, r = 0.99 or better). The comparison between groups were made using Student's t test at 95% confidence level.

**Table 1: Serum anti-HBsAg IgG antibody titers in Rats based on size of HBsAg entrapped polymer particle**

| **Time (Days)** | **Alum Adsorbed HBsAg** | **S.E.** | **HBsAg MP** | **S.E.** | **HBsAg MP + Alum** | **S.E.** | **HBsAg NP** | **S.E.** | **HBsAg NP + Alum** | **S.E.** |
|---|---|---|---|---|---|---|---|---|---|---|
| | **(mIU/ml)** | | **(mIU/ml)** | | **(mIU/ml)** | | **(mIU/ml)** | | **(mIU/ml)** | |
| 0 | 0 | 0 | 0 | 0 | 0 | 0 | 0 | 0 | 0 | 0 |
| 30 | 1368.25 | 918.18 | 328.52 | 150 | 1767.2 | 523.95 | 172.04 | 126.7 | 959.17 | 347.1 |
| 60 | 2649.5 | 100 | 598.3 | 160.9 | 3682.4 | 150 | 1009.8 | 191.2 | 1909.3 | 100 |
| 90 | 3998.6 | 150 | 1803.3 | 150 | 4940.9 | 100 | 757.4 | 307 | 2065.5 | 100 |
| 120 | 3649.9 | 100 | 1651.03 | 330.37 | 4435.07 | 237.96 | 699.59 | 150 | 1714.4 | 250.58 |
| 180 | 1398.31 | 399.3 | 907.42 | 0.43 | 1814 | 0.17 | 203.5 | 101.45 | 1276.7 | 731.64 |

| | | | | | | | | | | |
|---|---|---|---|---|---|---|---|---|---|---|
| MP: Microparticles; NP: Nanoparticles; S.E.: Standard error | | | | | | | | | | |

**Table 2: T-cell proliferation assay in BALB/c mice using different sized HBsAg entrapped particles in vivo**

| **Soluble HBsAg (Recall Ag)** | **HBsAg Nano (200nm)** | **S.E.** | **HBsAg Nano (400nm)** | **S.E.** | **HBsAg Micro (2-8 µm)** | **S.E.** | **Voluble HBsAg** | **S.E.** | **Control** | **S.E.** |
|---|---|---|---|---|---|---|---|---|---|---|
| **(µg/ml)** | **(CCPM)** | | **(CCPM)** | | **(CCPM)** | | **(CCPM)** | | **(CCPM)** | |
| 10 | 25077.5 | 171.5 | 51248.5 | 2273.5 | 48140 | 3681 | 3125 | 8 | 2852.5 | 339. 5 |
| 5 | 24954.5 | 4869.5 | 42301 | 5707 | 70341.5 | 2189.5 | 2379 | 48 | 1968.5 | 68.5 |
| 2.5 | 24841 | 1299 | 39426 | 0 | 32592 | 3192 | 3221.5 | 743.5 | 1890 | 153 |
| 1.25 | 26277 | 0 | 33030 | 0 | 32154 | 0 | 3369 | 307 | 1895 | 0 |
| 0.63 | 21797 | 0 | 15411 | 456 | 21823 | 0 | 3494.5 | 453.5 | 1895 | 0 |
| 0.31 | 15056 | 0 | 11335.5 | 1810.5 | 21159 | 0 | 3417 | 0 | 1895 | 0 |
| 0.15 | 10622 | 1416 | 7661.5 | 1091.5 | 22318 | 0 | 2652 | 125 | 1895 | 0 |
| 0.08 | 11922.5 | 4636.5 | 6308.5 | 176.5 | 22566 | 0 | 3085 | 568 | 1895 | 0 |

| | | | | | | | | | | |
|---|---|---|---|---|---|---|---|---|---|---|
| S.E.: Standard error; CCPM: Cell count per minute | | | | | | | | | | |

**Table 3: Cytokine assay for the quantitative analysis of mouse IFN-γ in culture supernatants from T-cell proliferation assay (top)**

| **Soluble HBsAg (Recall Ag)** | **HBsAg Nano (200nm)** | **HBsAg Nano (400nm)** | **HBsAg Micro (2-8 µm)** | **Soluble HBsAg** | **Control** |
|---|---|---|---|---|---|
| **(µg/ml)** | **(pg/ml)** | **(pg/ml)** | **(pg/ml)** | **(pg/ml)** | **(pg/ml)** |
| 10 | 474.9 | 718.20 | 153.87 | 99.75 | 94.69 |
| 5 | 227.07 | 1305.11 | 280.89 | 94.69 | 89.68 |
| 2.5 | 287.9 | 648.53 | 220.63 | 94.69 | 84.74 |
| 1.25 | 165.44 | 728.41 | 369.13 | 89.68 | 84.74 |
| 0.63 | 136.98 | 2569.89 | 246.79 | 99.75 | 0 |
| 0.31 | 136.98 | 1200.68 | 183.28 | 89.68 | 0 |
| 0.16 | 126.03 | 189.34 | 120.65 | 89.68 | 0 |
| 0.08 | 104.88 | 253.48 | 159.62 | 89.68 | 0 |

**Table 4: Cytokine assay for the quantitative analysis of mouse IL-4 in culture supernatants from T-cell proliferation assay (top)**

| **Soluble HBsAg (Recall Ag)** | **HBsAg Nano (200nm)** | **HBsAg Nano (400nm)** | **HBsAg Micro (2-8 µm)** | **Soluble HBsAg** | **Control** |
|---|---|---|---|---|---|
| **(µg/ml)** | **(pg/ml)** | **(pg/ml)** | **(pg/ml)** | **(pg/ml)** | **(pg/ml)** |
| 10 | 24.14 | 22.78 | 77.32 | 30.63 | 30.63 |
| 5 | 24.78 | 22.32 | 115.59 | 30.63 | 30.63 |
| 2.5 | 26.99 | 21.09 | 100.80 | 30.63 | 30.63 |
| 1.25 | 26.99 | 24.14 | 55.98 | 30.63 | 30.63 |
| 0.63 | 21.09 | 26.20 | 38.27 | 30.63 | 30.63 |
| 0.31 | 21.38 | 26.20 | 29.18 | 29.64 | 30.63 |
| 0.16 | 24.78 | 27.82 | 34.49 | 31.66 | 30.63 |
| 0.08 | 24.78 | 28.71 | 39.63 | 30.63 | 30.63 |

## Claims

1. Vaccine composition for use in the treatment of a disease or clinical manifestation involving the induction of a combined TH1 and TH2 immune response, the vaccine composition comprising an antigen being encapsulated in polymeric particles, wherein
- said polymeric particles comprise a combination of nanoparticles and microparticles;
- said polymeric particles are of a biodegradable polymer;
- the size of said nanoparticles ranges from 200 - 600 nm;
- the size of said microparticles ranges from 2 - 8 µm; and
- said nanoparticles induce a TH1 immune response and said microparticles induce a TH2 immune response.

2. The vaccine composition for use according to claim 1, wherein said nanoparticles and microparticles comprise the same antigen.

3. The vaccine composition for use according to claim 1 or 2, wherein said antigen is selected from the group consisting of proteins, polysaccharides, glycoproteins, glycolipids, nucleic acids, and combinations thereof.

4. The vaccine composition for use according to any one of claims 1 to 3, wherein said antigen is derived from an organism selected from the group consisting of rotavirus, paramyxovirus, mumps virus, rubella virus, polio virus, hepatitis virus, herpes viruses, human immunodeficiency virus, *Haemophilus influenza, Clostridium tetani, Corynebacterium diphtheria,* and *Neisseria gonorrhea.*

5. The vaccine composition for use according to claim 4, wherein said antigen is selected from the group consisting of hepatitis B surface antigen, tetanus toxoid, and diphtheria toxin.

6. The vaccine composition for use according to any one of claims 1 to 5, wherein said biodegradable polymer is selected from the group consisting of poly (lactide-co-glycolide), polylactide, and polyglycolide.

7. The vaccine composition for use according to claim 6, wherein said biodegradable polymer is polylactide polymer.

8. The vaccine composition for use according to any one of claims 1 to 7, wherein the disease is selected from the group consisting of malaria, tuberculosis, hepatitis, diphtheria, tetanus, cancer, and human immunodeficiency virus infection.

9. The vaccine composition for use according to any one of claims 1 to 8, further comprising the modulation of the TH1 and TH2 immune responses from the same antigen.

10. The vaccine composition for use according to claim 9 wherein the modulation further comprises promoting TH1 and TH2 immune responses in a balanced manner.

11. The vaccine composition for use according to any one of claims 1 to 10, wherein said composition is used as a single-shot vaccine formulation.

## Patentansprüche

1. Impfstoffzusammensetzung zur Verwendung bei der Behandlung einer Erkrankung oder klinischen Manifestation, die mit der Induktion einer kombinierten TH1 und TH2 Immunantwort einhergeht, wobei die Impfstoffzusammensetzung ein Antigen umfasst, das in polymeren Partikeln verkapselt ist, wobei
- die polymeren Partikel eine Kombination aus Nanopartikeln und Mikropartikeln umfassen;
- die polymeren Partikel aus einem biologisch abbaubaren Polymer bestehen;
- die Größe der Nanopartikel zwischen 200 nm und 600 nm liegt;
- die Größe der Mikropartikel zwischen 2 µm und 8 µm liegt; und
- die Nanopartikel eine TH1 Immunantwort induzieren, und die Mikropartikel eine TH2 Immunantwort induzieren.

2. Impfstoffzusammensetzung zur Verwendung gemäß Anspruch 1, wobei die Nanopartikel und Mikropartikel das gleiche Antigen umfassen.

3. Impfstoffzusammensetzung zur Verwendung gemäß Anspruch 1 oder 2, wobei das Antigen aus der Gruppe ausgewählt wird, die aus Proteinen, Polysacchariden, Glycoproteinen, Glycolipiden, Nukleinsäuren und Kombinationen davon besteht.

4. Impfstoffzusammensetzung zur Verwendung gemäß einem der Ansprüche 1 bis 3, wobei das Antigen von einem Organismus abgeleitet ist, der aus der Gruppe ausgewählt wird, die aus Rotavirus, Paramyxovirus, Mumpsvirus, Rubellavirus, Poliovirus, Hepatitisvirus, Herpesvirus, humanem Immundefizienzvirus, *Haemophilus influenza, Clostridium tetani, Corynebacterium diphtheriae* und *Neisseria gonorrhea* besteht.

5. Impfstoffzusammensetzung zur Verwendung gemäß Anspruch 4, wobei das Antigen aus der Gruppe ausgewählt wird, die aus dem Hepatitis B Oberflächenantigen, Tetanus Toxoid und Diphtherietoxin besteht.

6. Impfstoffzusammensetzung zur Verwendung gemäß einem der Ansprüche 1 bis 5, wobei das biologisch abbaubare Polymer aus der Gruppe ausgewählt wird, die aus Poly(lactid-co-glycolid), Polylactid und Polyglycolid besteht.

7. Impfstoffzusammensetzung zur Verwendung gemäß Anspruch 6, wobei das biologisch abbaubare Polymer ein Polylactidpolymer ist.

8. Impfstoffzusammensetzung zur Verwendung gemäß einem der Ansprüche 1 bis 7, wobei die Erkrankung aus der Gruppe ausgewählt wird, die aus Malaria, Tuberkulose, Hepatitis, Diphtherie, Tetanus, Krebs und einer Infektion mit dem humanen Immundefizienzvirus besteht.

9. Impfstoffzusammensetzung zur Verwendung gemäß einem der Ansprüche 1 bis 8, die ferner die Modulation der TH1 und TH2 Immunantworten des gleichen Antigens umfasst.

10. Impfstoffzusammensetzung zur Verwendung gemäß Anspruch 9, wobei die Modulation ferner die Förderung der TH1 und TH2 Immunantworten in einer ausgewogenen Weise umfasst.

11. Impfstoffzusammensetzung zur Verwendung gemäß einem der Ansprüche 1 bis 10, wobei die Zusammensetzung als Einzel (single-shot)-Impfstoffformulierung verwendet wird.

## Revendications

1. Composition de vaccin pour une utilisation dans le traitement d'une maladie ou les manifestations cliniques comportant l'induction d'une réponse immunitaire combiné TH1 et TH2, la composition de vaccin comprenant un antigène est encapsulé dans des particules polymériques, dans lequel
- lesdites particules polymères comprennent une combinaison de nanoparticules et de microparticules;
- lesdites particules polymères sont d'un polymère biodégradable;
- la taille desdites plages nanoparticules de 200-600 nm;
- la taille desdites plages microparticules 2-8 µm; et
- lesdites nanoparticules induire une réponse immunitaire Th1 et lesdites microparticules induire une réponse immunitaire Th2.

2. Composition de vaccin pour usage selon la revendication 1, dans lequel lesdites nanoparticules et des microparticules comprennent le même antigène.

3. Composition de vaccin pour usage selon la revendication 1 ou 2, dans lequel ledit antigène est choisi dans le groupe constitué par les protéines, les polysaccharides, les glycoprotéines, glycolipides, des acides nucléiques et leurs combinaisons.

4. Composition de vaccin pour une utilisation selon l'une quelconque des revendications 1 à 3, dans lequel ledit antigène est dérivée d'un organisme choisi dans le groupe constitué par le rotavirus, des paramyxovirus, le virus des oreillons, virus de la rubéole, le virus de la polio, le virus de l'hépatite, virus de l'herpès, d'immunodéficience humaine virus, infections à *Haemophilus influenzae, Clostridium tetani, Corynebacterium diphteriae* et *Neisseria gonorrhoeae.*

5. Composition de vaccin pour usage selon la revendication 4, dans lequel ledit antigène est choisi dans le groupe constitué de l'antigène de l'hépatité B, l'anatoxine tétanique, et la toxine diphtérique.

6. Composition de vaccin pour une utilisation selon l'une quelconque des revendications 1 à 5, dans lequel ledit polymère biodégradable est choisi dans le groupe constitué par le poly (lactide-co-glycolide), polylactides, et polyglycolide.

7. Composition de vaccin pour usage selon la revendication 6, dans lequel ledit polymère biodégradable est un polylactide polymère.

8. Composition de vaccin pour une utilisation selon l'une quelconque des revendications 1 à 7, dans laquelle la maladie est choisie dans le groupe constitué par la malaria, la tuberculose, l'hépatite, la diphtérie, le tétanos, le cancer, et une infection virus de l'immunodéficience humaine.

9. Composition de vaccin pour une utilisation selon l'une quelconque des revendications 1 à 8, comprenant en outre la modulation des réponses immunitaires TH1 et TH2 du même antigène.

10. Composition de vaccin pour usage selon la revendication 9, dans lequel la modulation comprend en outre la promotion TH1 et TH2 réponses immunitaires d'une manière équilibrée.

11. Composition de vaccin pour une utilisation selon l'une quelconque des revendications 1 à 10, dans lequel ladite composition est utilisée comme une formulation de vaccin à un coup.
